# EUROPEAN PATENT APPLICATION

(11) **EP 1 576 964 A1**
(43) Date of publication of application: **21.09.2005**
(21) Application number: 03775875.2
(22) Date of filing: 25.11.2003
(51) Int. Cl.: A61K 38/40, A61K 38/17, A61P 3/14, A61P 19/10, A61P 43/00

(54) **PROTEASE INHIBITOR**

(30) Priority: 29.11.2002 JP 2002347802
(71) Applicant: MORINAGA MILK INDUSTRY CO., LTD., Minato-ku, Tokyo 108-8384 (JP)
(72) Inventor: KATUNUMA, Nobuhiko, c/o TOKUSHIMA BUNRI UNIVERSITY, Yamashiro-cho, Tokushima-shi, Tokushima (JP)
(74) Representative: Bannerman, David Gardner
(86) International application number: PCT/JP2003/015007
(87) International publication number: WO 2004/050116

(57) **Abstract**

This invention relates to a cysteine protease inhibitor comprising one or more of lactoferrin, a partial peptide of lactoferrin, and transferrin, as an active ingredient. The cysteine protease inhibitor of the present invention can be used for preventive or therapeutic drug for osteoporosis, malignant hypercalcemia etc. and used for food, drink, feed and the like.

## Description

### Technical Field

The present invention relates to a cysteine protease inhibitor comprising one or more of components selected from the group consisting of lactoferrin, a partial peptide of lactoferrin or transferrin as an active ingredient, which can be used for preventive or therapeutic agents for osteoporosis, malignant hypercalcemia etc., and used for food, drink, feed and the like.

### Background Art

A proteolytic enzyme having a thiol group at its active center is generically called as cysteine protease (thiol protease). Cathepsin L, cathepsin B or cathepsin K is one of typical cysteine proteases along with calcium-dependent neutral protease (CAMP), papain, ficin, promelain and the like. Substances having inhibitory action against those cysteine proteases are expected to be used as therapeutic agents for diseases associated with cysteine proteases including muscular dystrophy, dystrophia, myocardial infarct, apoplexy, Alzheimer's disease, disturbance of consciousness or motility caused by head trauma, multiple sclerosis, peripheral neuropathy, cataract, inflammation, allergy, fulminant hepatitis, osteoporosis, hypercalcemia, breast cancer, prostate cancer or enlarged prostate, or as a growth inhibitor or metastasis preventive agent for cancer, an antithrombotic drug and the like. Moreover, in recent years, the relationship between cathepsin L, cathepsin B, and osteoporosis or malignant hypercalcemia has been elucidated based on the studies made by Katsunuma et al. Accordingly, in particular, application of a cathepsin L inhibitor as a therapeutic agent for osteoporosis or malignant hypercalcemia is attracting attention (Nobuhiko Katsunuma, "BIO media" Vol. 7, No. 6, pp. 73 to 77, 1992). In bone tissue, osteogenesis by osteoblast and bone resorption by osteoclast are occurring throughout life. In growing ages, bone mass is increased due to excessive osteogenesis over bone resorption, whereas in older ages, bone mass is decreased due to excessive bone resorption over osteogenesis, which leads to development of osteoporosis. There are various causes of osteoporosis, and especially, one of main causes thereof is bone collapse (bone resorption), which is further divided into the following two causes. That is, one is attributed to failure of absorption and deposition of calcium, more specifically related to supply, transfer, absorption and deposition of calcium, in which vitamin D derivatives, female hormone (estrogen) and the like are considered to be involved. The other one is associated with promoted degradation of collagen, a bone-supporting tissue, and a principal cause thereof is degradation of bone collagen by cysteine proteases which are secreted from the lysosome located in the osteoclast, in particular, cathepsin L, cathepsin B and cathepsin K. The cathepsin L and capthepsin B secreted from the lysosome in the osteoclast promote degradation of collagen in bone tissue, whereby old bones are caused to lyse, and calcium is freed and released into the blood together with hydroxyproline. Therefore, inhibition of cathepsin L, cathepsin B and cathepsin K-mediated collagen degradation enables prevention of excessive bone collapse, thus making it possible to treat osteoporosis. Estrogen, anabolic hormone, calcium preparation, vitamin D, calcitonin, bisphosphonate or the like is known as a therapeutic agent for such osteoporosis. In addition, development of a therapeutic agent for osteoporosis using some cysteine protease inhibitors is under progress as regards a therapeutic agent for osteoporosis having a mechanism of action of so-called cysteine protease inhibition including cathepsin L inhibition, cathepsin B inhibition and cathepsin K inhibition (JP 07-179496 A, JP 2002-501502A). However, further development of therapeutic agents for osteoporosis has been desired.

Meanwhile, hypercalcemia is a metabolic disorder where calcium concentration in serum is elevated beyond the normal value, and is often seen in patients with tumor. It is said that, if hypercalcemia is neglected, the life of the patients would be 10 days long at most. In many cases, it is caused due to bone metastasis of tumor. When tumor transfers to bone, bone collapse occurs and calcium is released into the blood. The released calcium is disposed in the kidney, and hypercalcemia develops when speed of bone collapse exceeds the disposing capacity of the kidney. As a method of treatment, a method of promoting calcium excretion from the kidney by use of infusion of physiological saline with furosemide and a method of using calcitonin as a therapeutic agent for osteoporosis are known. Namely, it is said that a therapeutic agent for osteoporosis which suppresses bone resorption can be also effective as a therapeutic agent for malignant hypercalcemia.

The followings have already been disclosed as cysteine protease inhibitors which may be used for such purposes, by the inventors of the present invention.
(1) Cathepsin L-specific inhibitory polypeptide (JP 07-179496 A)
(2) Thiol protease inhibitor (JP 09-221425 A)
(3) Valine derivative and its use (JP 2001-139534 A)
(4) Thiol protease inhibitor (JP 07-242600 A)
(5) FA-70C1 substance (JP 2000-72797 A)
(6) FA-70D substance, its production method and its use (WO 97/31122)

However, further development of cysteine protease inhibitors which are antigen-free and can be used as safe material has been desired.

On the other hand, it has been known so far that protease inhibitory substances are present in breast milk. The known protease inhibitory substances contained in breast milk include α1-antichymotrypsin and α1-antitrypsin, and inhibitors including inter α2-trypsin inhibitory substance, α2-antiplasmin, α2-macroglobulin, antithrombin III and antileukoprotease are contained in a minute amount in breast milk (Isao Kiyosawa, "Human Milk in Infant Nutrition" Kanehara & Co., Ltd., pp. 80 to 81).

The following proteins having cysteine protease inhibitory activity in milk have already been disclosed.
(1) Novel cysteine protease inhibitor with a molecular weight of approximately 57 kDa which has a sugar chain and is derived from bovine colostrum (JP 07-2896 A).
(2) Novel cysteine protease inhibitor with a molecular weight of 16 ± 2 kDa or 13 ± 2 kDa which is derived from colostrum (JP 07-126294 A).
(3) Novel protein with a molecular weight of 16 ± 2 kDa or 13 ± 2 kDa which is derived from human milk, and a production method for the same (JP 10-80281 A).
(4) Bone resorption inhibitor which includes basic cystatin of milk origin which is prepared from milk and/or degradation product of the basic cystatin of milk origin, as an active ingredient (JP 2000-281587 A).
(5) Cystatin C contained in milk basic protein (MBP), and a bone resorption inhibitory effect by the cystatin C in vitro ("Bioscience, Biotechnology, and Biochemistry, Japan" Vol. 66, No. 12, 2002, pp. 2531 to 2536).

Proteins contained in mammalian milk in a large amount include lactoferrin and β-casein. Lactoferrin (hereinafter, sometimes abbreviated to Lf) is an iron-bound glycoprotein which is mainly contained in breast milk and has molecular weight of approximately 80kDa, and it is known to exhibit antibacterial effects on pathogenic microorganisms including Escherichia coli, Candida, Clostridium and Staphylococcus (Journal of Pediatrics, vol. 94, page 1, 1979 and Journal of Dairy Science, vol. 67, page 60, 1984). Further, it is widely used for treatment of diseases, as a milk protein having various activities. Lactoferrin, a protein derived from milk, has high safety level and can be continuously used for a long term. Further, lactoferrin itself has no flavor and no smell and therefore, it is versatile as an additive into various foods, pharmaceuticals and feed.

As examples of therapeutic agents to which lactoferrin is applied, the followings are disclosed.
(1) Anti-tumor agent (JP 05-86932 B)
(2) Anti-rheumatoid agent (JP 05-186368 A)
(3) IgA production-accelerating agent (JP 06-32743 A)
(4) Processed milk powder for baby nursing effective for improving state of feces.(JP 06-205640 A)
(5)Therapeutic agent for angiogenesis-related disease (JP09-194388 A)
(6) Oral agent for inhibiting cancer metastasis (JP 10-59864)
(7) IgE production-inhibitor (JP 2001-158748 A)
   As effects of hydrolysate of Lf, the followings are disclosed.
(8) Antibacterial effect and tyrosinase-inhibitory effect (European Patent 438750)
(9) Inhibitory effect on attachment of pathogenic bacteria to cells (JP 03-220130 A)
(10) Antivirus effect (JP 01-233226 A)

Further, it is disclosed that bone-enriching agent comprising iron-bound lactoferrin as an active ingredient is effective for preventing or treating bone-related diseases (JP 2000-281586 A). This is a technology where iron, a coenzyme of an enzyme involved in hydration of proline and lysine which are precursors of bone substrate collagen, is provided by administering iron-lactoferrin, namely, iron-bound lactoferrin, as an active ingredient, and thereby, collagen-synthesis is enhanced.

Asdescribe above, cysteine protease-inhibitory substances have been thus far found and they have also been found in mammalian cells, blood, urine and milk. Among cysteine protease-inhibitory substances, substances derived from proteins are generally called cystatin. It is known that the cystatin family has common active site, and the sequence is disclosed (Hayaishi Osamu wrote, "proteases and inhibitors thereof," Medical View Co. Ltd. Eds., 1^{st} edition, 1^{st} printing, pages 104-115, 1993).

However, it has not been known that lactoferrin, partial peptide thereof and transferrin have cysteine protease inhibitory activity. Further, neither had it been known that lactoferrin and transferrin have a region homologous to the sequence of the active site preserved in cystatin family. Moreover, agents for preventing or treating bone-related diseases, which have a mechanism of action of inhibiting cystein protease activity by lactoferrin, partial peptide thereof or transferrin, has not been known.

### Disclosure of the Invention

An object of the present invention is to provide a versatile cysteine protease inhibitor which can be widely used as foodmaterials, and which can be used for preventive or therapeutic agents for osteoporosis, malignant hypercalcemia and the like, and used for various types of food, drink and feed.

As a result of extensive studies for searching a cysteine protease inhibitor which may be used as antigenic-free and safe material, the inventors of the present invention found that lactoferrin which is a protein derived from milk, a peptide fragment derived from lactoferrin, and transferrin have cysteine protease inhibitory activity, and thereby completed the present invention.

The gist of the present invention is as in the following (1) to (12).
(1) A cysteine protease inhibitor which comprises one or more of lactoferrin, a partial peptide of lactoferrin, and transferrin, as an active ingredient.
(2) The cysteine protease inhibitor according to (1), wherein lactoferrin and/or transferrin are one or more of metal-saturated form, partially metal-saturated form and apo form.
(3) The cysteine protease inhibitor according to (1) or (2), wherein lactoferrin and/or transferrin are one or more of the proteins or peptides as shown in the following (a) to (d);
   (a) a protein or peptide having amino acid sequence of at least amino acid numbers 679 to 695 of the amino acid sequence shown in SEQ ID No. 1 of the Sequence Listing,
   (b) a protein or peptide having amino acid sequence of at least amino acid numbers 679 to 695 of the amino acid sequence shown in the SEQ ID No. 1 of the Sequence Listing, including substitution, deletion, addition or inversion of one or plural amino acids, and having cysteine protease inhibitory activity,
   (c) a protein or peptide having amino acid sequence of at least amino acid numbers 676 to 692 of the amino acid sequence shown in SEQ ID No. 2 of the Sequence Listing, and
   (d) a protein or peptide having amino acid sequence of at least amino acid numbers 676 to 692 of the amino acid sequence shown in the SEQ ID No. 2 of the Sequence Listing, including substitution, deletion, addition or inversion of one or plural amino acids, and having cysteine protease inhibitory activity.
(4) The cysteine protease inhibitor according to any one of (1) to (3), which is a preventive or therapeutic agent for a disease associated with cysteine protease.
(5) The cysteine protease inhibitor according to (4), wherein the disease associated with cysteine protease is osteoporosis or malignant hypercalcemia.
(6) A food and drink composition or feed composition, which is produced by adding the cysteine protease inhibitor according to any one of (1) to (5).
(7) A method for treating a disease associated with cysteine protease, wherein the cysteine protease inhibitor according to any one of (1) to (6) is administered to a subject.
(8) A use of one or more of lactoferrin, a peptide fragment of lactoferrin, and transferrin in manufacture of a cysteine protease inhibitor.
(9) The use according to (8), wherein lactoferrin and/or transferrin are one or more of metal-saturated form, partially metal-saturated form and apo form.
(10) The use according to (8) or (9), wherein lactoferrin and/or transferrin are one or more of the proteins or peptides as shown in the following (a) to (d);
   (a) a protein or peptide having amino acid sequence of at least amino acid numbers 679 to 695 of the amino acid sequence shown in SEQ ID No. 1 of the Sequence Listing,
   (b) a protein or peptide having amino acid sequence of at least amino acid numbers 679 to 695 of the amino acid sequence shown in the SEQ ID No. 1 of the Sequence Listing, including substitution, deletion, addition or inversion of one or plural amino acids, and having cysteine protease inhibitory activity,
   (c) a protein or peptide having amino acid sequence of at least amino acid numbers 676 to 692 of the amino acid sequence shown in SEQ ID No. 2 of the Sequence Listing, and
   (d) a protein or peptide having amino acid sequence of at least amino acid numbers 676 to 692 of the amino acid sequence shown in the SEQ ID No. 2 of the Sequence Listing, including substitution, deletion, addition or inversion of one or plural amino acids, and having cysteine protease inhibitory activity.
(11) The use according to any one of (8) to (10), wherein the cysteine protease inhibitor is a preventive and therapeutic agent for a disease associated with cysteine protease.
(12) The use according to (11), wherein the disease associated with cysteine protease is osteoporosis or malignant hypercalcemia.

### Brief Description of the Drawings

Fig. 1 is a diagram (photograph) which shows detection of bovine milk protein by reverse zymography.
Fig. 2 is a diagram which shows inhibitory activities of human lactoferrin to cysteine proteases.

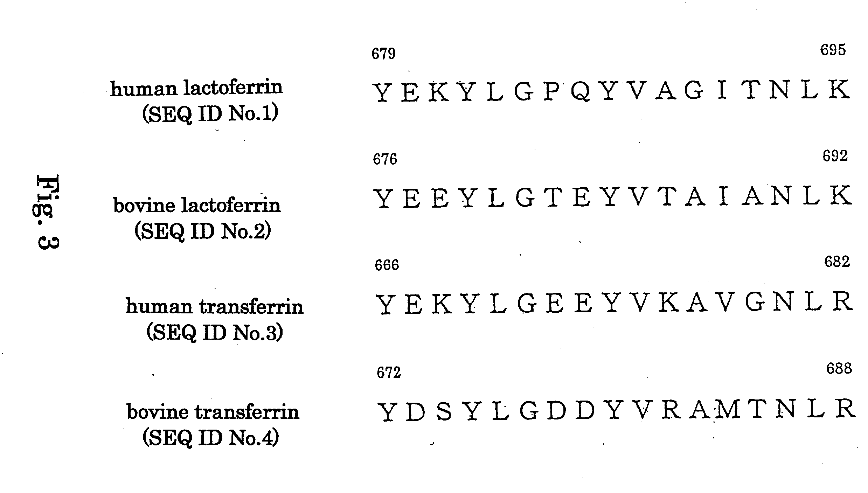

Fig. 3 is a diagram which shows amino acid sequences of a partial peptide of human lactoferrin (human lactoferrin peptide Y679-K695) which was produced in Production Example 1, a partial peptide of bovinelactoferrin (bovinelactoferrinpeptideY676-K692), a peptide having amino acid sequence of amino acid numbers 666-682 in the amino acid sequence shown in SEQ ID No. 3 of the Sequence Listing (human transferrin peptide Y666-R682) and a peptide having amino acid sequence of amino acid numbers 672-688 in the amino acid sequence shown in SEQ ID No. 4 of the Sequence Listing (bovine transferrin peptide Y672-R688).
Fig. 4 is a diagram which shows inhibitory activities of human lactoferrin peptide Y679-K695 to cysteine proteases.
Fig. 5 is a diagram which shows inhibitory activities of transferrin to cysteine proteases.

### Best Mode for carrying out the Invention

Preferred embodiments of the present invention will be described in detail below. However, the present invention is not limited to the following preferred embodiments, and any changes may be made within the scope of the present invention. All percentages used in the present specification are represented by mass unless otherwise noted.

The present invention relates to a cysteine protease inhibitor comprising one or more of lactoferrin (e.g., human lactoferrin having amino acid sequence of Swiss-Prot Accession No.:P02788, bovine lactoferrin having amino acid sequence of Swiss-Prot Accession No.:P24627, goat lactoferrin having amino acid sequence of Swiss-Prot Accession No.:Q29477, horse lactoferrin having amino acid sequence of Swiss-Prot Accession No.:077811), a partial peptide of lactoferrin, transferrin (e.g., human transferrin having amino acid sequence of Swiss-Prot Accession No.:P02787,bovine transferrin having amino acid sequence of Swiss-Prot Accession No.:Q29443, horse transferrin having amino acid sequence of Swiss-Prot Accession No.:P27425), as an active ingredient.

Lactoferrin used in the present invention may be commercially available lactoferrin, or lactoferrin which can be isolated according to conventional procedures including ion-exchange chromatography from raw materials such as mammalian colostrum, transitional milk, matured milk, last-phase milk, or skim milk or whey, which are processed milk. In particular, it is preferable to use commercially available lactoferrin (e.g. manufactured by Morinaga Milk Industry Co., Ltd.) which is produced at an industrial scale. In addition, lactoferrin produced by genetic engineering using microorganisms, mammalian cells or transgenic animals etc. can be used.

Metal content in lactoferrin is not particularly limited for the effect of the cystein protease inhibitor of the present invention, and one or more of the groups consisting of apo-form lactoferrin which is deironized with hydrochloric acid or citric acid etc., metal-saturated lactoferrin having saturation degree of 100% which is obtainable by chelating the apo-form lactoferrin with metals including iron, copper, zinc, manganese etc., and lactoferrin partially saturated with metal in which metal is bound at saturation degree of less than 100% can be used.

As production methods of partial peptide of lactoferrin used in the present invention, there can be exemplified a production method by hydrolyzing the above-mentioned lactoferrin with acid or protease according to conventional procedures, a production method by producing recombinant peptides according to genetic engineering or a method of producing synthetic peptides according to chemical synthesis. When the production method by hydrolysis is applied, the partial peptide of lactoferrin of the present invention can be used as a mixture in which partial peptide is contained in hydrolysate, or can be used as a peptide purified according to conventional procedures including HPLC etc.

Preferable forms of lactoferrin or partial peptide of lactoferrin used in the present invention can be exemplified as a human lactoferrin having amino acid sequence shown in SEQ ID No.1 of the Sequence Listing or protein or peptide having amino acid sequence of at least amino acid numbers 679-695 of the amino acid sequence shown in SEQ ID No.1 of the Sequence Listing. There can also be exemplified a bovine lactoferrin having amino acid sequence shown in SEQ ID No.2 of the Sequence Listing or protein or peptide having amino acid sequence of at least amino acid numbers 676-692 of the amino acid sequence shown in SEQ ID No.2 of the Sequence Listing. Note that sequences of amino acid numbers 1 to 19 of the amino acid sequence shown in SEQ ID No. 1 of the Sequence Listing and the amino acid sequence shown in SEQ ID No. 2 of the Sequence Listing are signal sequences.

These lactoferrin and partial peptide of lactoferrin have cysteine protease inhibitory activity, and therefore they can be used for the cysteine protease inhibitor of the present invention.

Moreover, it is considered that peptides having amino acid sequence which includes amino acid numbers 679 to 695 of SEQ ID No. 1 in the Sequence Listing and further extends to one or both of the N-termius side and the C-termius side, and peptides having amino acid sequences which includes amino acid numbers 676 to 692 of SEQ ID No. 2 in the Sequence Listing and further extends to one or both of the N termius side and the C termius side, have cysteine protease inhibitory activity, because full-length lactoferrin has cysteine protease inhibitory activity as described later.

Since the present invention have revealed a domain showing cysteine protease inhibitory activity, the above proteins or peptides may be obtained by chemical synthesis based on amino acid sequence including the domain, as well as by gene recombination techniques. For example, appropriate primers are prepared on the basis of the nucleotide sequence coding for the amino acid sequence including the domain. The nucleotide sequence is then amplified by PCR and the like using the primers and cDNA including the target nucleotide sequence as a template. The obtained nucleotide sequence is expressed using an appropriate expression system, thereby the proteins or peptides described above can be obtained.

In general, there exist mutations including substitution, deletion, insertion, addition or inversion of one or plural nucleotides at one or plural positions in a gene, due to difference in species, genus, individual and the like, and substitution arises in an amino acid of a protein encoded by a gene including the above mutation. Lactoferrin and a partial peptide of lactoferrin which can be used for the present invention may thus include such substitution within a range that the cysteine protease inhibitory activity is not impaired.

Lactoferrin or a partial peptide of lactoferrin which can be used for the present invention includes a protein or peptide having amino acid sequence of at least amino acid numbers 679 to 695 of the amino acid sequence shown in SEQ ID No. 1 of the Sequence Listing or a protein or peptide having amino acid sequence of at least amino acid numbers 676 to 692 of the amino acid sequence shown in SEQ ID No. 2 of the Sequence Listing, which includes substitution, deletion, insertion, addition, or inversion of one or plural amino acids and has cysteine protease inhibitory activity. "Substitution, deletion, insertion, addition or inversion of one or plural amino acids" may be set arbitrary as long as it does not influence steric interaction between the protein or the peptide having amino acid sequence of the above-mentioned amino acid numbers and cysteine protease, and does not impair cysteine protease activity. For example, "plural" refers to 2 to 5 amino acids, preferably 2 or 3 amino acid, more preferably 2 amino acids. It varies with a position or a kind of an amino acid residue in the protein conformation of amino acid numbers 679 to 695 of the amino acid sequence shown in SEQ ID No. 1 of the Sequence Listing, or amino acid numbers 676 to 692 of the amino acid sequence shown in SEQ ID No. 2 of the Sequence Listing.

Substitution in the amino acid of amino acid numbers 686 to 690 of the amino acid sequence shown in SEQ ID No. 1 of the Sequence Listing or in the amino acid of amino acid numbers 683 to 687 of the amino acid sequence shown in SEQ ID No. 2 of the Sequence Listing is preferably a substitution which does not change the kind of amino acid. Namely, it is preferable that the replacing amino acids in the above-mentioned sequence are the amino acid having the similar character in the structural classification as the replaced amino acid. Specifically, replacement by aspartic acid, glutamic acid, asparagine or glutamine are preferable when the amino acid in the above-mentioned sequence is acidic amino acid or amide of acidic amino acid, replacement by lysine, histidine or arginine are preferable in the case of basic amino acid, replacement by phenylalanine, tyrosine or tryptophan is preferable in the case of aromatic amino acid, replacement by glycine, alanine, valine, leucine, isoleucine, serine or threonine is preferable in the case of aliphatic amino acid or oxyamino acid, further, in the case of amino acid other than the above-mentioned amino acids (cysteine, methionine, proline etc.), it is preferable that replacement is performed arbitrary as long as it does not impair cysteine protease activity. Further, substitution, deletion and the like of one or plural amino acids may be included in amino acids except the amino acids of amino acid numbers 679 to 695 of the amino acid sequence shown in SEQ ID No. 1 of the Sequence Listing, or amino acids except the amino acids of amino acid numbers 676 to 692 of the amino acid sequence shown in SEQ ID No. 2 of the Sequence Listing. In this case, for example, the term plural refers to 2 to 10 amino acids, preferably 2 to 5 amino acids, more preferably 2 or 3 amino acids, though varying with a position or a kind of an amino acid residue in protein conformation.

Moreover, examples of lactoferrin or a partial peptide of lactoferrin which can be used for the present invention include a protein or a peptide which has homology not less than 80%, preferably not less than 90%, more preferably not less than 95% in an amino acid sequence with a protein or a peptide having amino acid sequence of at least amino acid numbers 679 to 695 of the amino acid sequence shown in SEQ ID No. 1 of the Sequence Listing, or a protein or a peptide having amino acid sequence of at least amino acid numbers 676 to 692 of the amino acid sequence shown in SEQ ID No. 2 of the Sequence Listing, and which has cysteine protease inhibitory activity.

Nucleotide sequence which codes for a protein or a peptide substantially identical with the lactoferrin protein or partial peptide of lactoferrin as described above can be obtained, for example, by modifying the nucleotide sequence with site-directed mutagenesis in such a manner that an amino acid residue located at a specific site includes substitution, deletion, insertion, addition or inversion. The modified nucleotide sequence may be obtained by means of conventional mutagenesis treatment.

Nucleotide sequence which codes for a protein or a peptide substantially identical with lactoferrin or partial peptide of lactoferrin may be obtained by expressing a nucleotide sequence including mutation in appropriate cells and examining cysteine protease inhibitory activity by a method of determining the cysteine protease inhibitory activity described in Test Examples or Examples of the present invention.

In the present invention, transferrin or partial peptide of transferrin may be used. The transferrin used in the present invention may be commercially available transferrin, or transferrin which can be obtained by isolating according to conventional procedures including column chromatography from raw materials including mammalian milk or blood. In addition, transferrin produced by genetic engineering using microorganisms, mammalian cells or transgenic animals etc. can be used.

Metal content in transferrin is not particularly limited for the effect of the cystein protease inhibitor of the present invention, and one or more of the groups consisting of apo-form transferrin which is deironized with hydrochloric acid or citric acid etc., metal-saturated transferrin having saturation degree of 100% which is obtainable by chelating the apo-form transferrin with metals including iron, copper, zinc, manganese etc., and transferrin partially saturated with metal in which metal is bound at saturation degree of less than 100% can be used.

In the present invention, partial peptide of transferrin may be used. The amino acid sequence of transferrin was compared with the amino acid sequences deduced to be the region having cysteine protease inhibitory activity of lactoferrin (the region of amino acid numbers 679-695 of SEQ ID No.1 or amino acid numbers 676-692 of SEQ ID No.2), and it was found that the region of amino acid numbers 666-682 (human transferrin peptide Y666-R682) of the amino acid sequence of human transferrin shown in SEQ ID No.3 and the region of amino acid numbers 672-688 (bovine transferrin peptide Y672-R688) of the amino acid sequence of bovine transferrin shown in SEQ ID No.4 have high homology with the region having cysteine protease inhibitory activity of lactoferrin (Figure 3). In addition to this fact, it was demonstrated that transferin have cysteine protease inhibitory activity as shown in the Examples described later, and therefore it was strongly suggested that partial peptides of human transferrin and bovine transferrin having the above-mentioned regions have cysteine protease inhibitory activity.

As production methods of partial peptide of transferrin used in the present invention, there can be exemplified a production method by hydrolyzing the above-mentioned transferrin with acid or protease according to conventional procedures, a production method by producing recombinant peptides according to genetic engineering or a method of producing synthetic peptides according to chemical synthesis. When the production method by hydrolysis is applied, partial peptide of transferrin of the present invention can be used as a mixture in which partial peptide is contained in hydrolysate, or can also be used as a peptide purified according to conventional procedures including HPLC etc.

Preferable forms of transferrin or partial peptide of transferrin used in the present invention can be exemplified as a human transferrin having amino acid sequence shown in SEQ ID No.3 of the Sequence Listing or a protein or peptide having amino acid sequence of at least amino acid numbers 666-682 of the amino acid sequence shown in SEQ ID No.3 of the Sequence Listing. There can also be exemplified bovine transferrin having amino acid sequence shown in SEQ ID No.4 of the Sequence Listing or a protein or peptide having amino acid sequence of at least amino acid numbers 672-688 of the amino acid sequence shown in SEQ ID No.4 of the Sequence Listing. Note that sequences of amino acid numbers 1 to 19 of the amino acid sequence shown in SEQ ID No. 3 of the Sequence Listing and the amino acid sequence shown in SEQ ID No. 4 of the Sequence Listing are signal sequences.

The peptide consisting of the amino acid sequence of amino acid numbers 666-682 of the amino acid sequence shown in SEQ ID No.3 of the Sequence Listing and the peptide consisting of the amino acid sequence of amino acid numbers 672-688 of the amino acid sequence shown in SEQ ID No.4 of the Sequence Listing are considered to have cysteine protease inhibitory activity based on the sequence homology with the amino acid sequence of amino acid numbers 679-695 of SEQ ID No.1 (human lactoferrin) of the Sequence Listing and the amino acid sequence of the amino acid numbers 676-692 of SEQ ID No. 2 (bovine lactoferrin) of the Sequence Listing, which are revealed to be regions with cysteine protease inhibitory activity in the present invention. Moreover, it is considered that peptides having amino acid sequence which includes amino acid numbers 666 to 682 of SEQ ID No. 3 in the Sequence Listing and further extends to one or both of the N-termius side and the C-termius side, and peptides having amino acid sequences which includes amino acid numbers 672 to 688 of SEQ ID No. 4 in the Sequence Listing and further extends to one or both of the N termius side and the C termius side, have cysteine protease inhibitory activity, because full-length transferrin has cysteine protease inhibitory activity.

Since the present invention have revealed a domain showing cysteine protease inhibitory activity, the above peptides may be obtained by chemical synthesis based on amino acid sequence including the domain, as well as by gene recombination techniques. For example, appropriate primers are prepared on the basis of the nucleotide sequence coding for amino acid sequence including the domain. The nucleotide sequence is then amplified by PCR and the like using the primers and cDNA including the target nucleotide sequence as a template. The obtained nucleotide sequence is expressed using an appropriate expression system, thereby the protein or peptide described above can be obtained.

In general, there exist mutations including substitution, deletion, insertion, addition or inversion of one or plural nucleotides at one or plural positions in a gene, due to difference in species, genus, individual and the like, and substitution arises in an amino acid of a protein encoded by a gene including the above mutation. Transferrin and a partial peptide of transferrin which can be used for the present invention may thus include such substitution within a range that the cysteine protease inhibitory activity is not impaired.

Transferrin or a partial peptide of transferrin which can be used for the present invention includes a peptide having amino acid sequence of at least amino acid numbers 666 to 682 of the amino acid sequence shown in SEQ ID No. 3 of the Sequence Listing or a peptide having amino acid sequence of at least amino acid numbers 672 to 688 of the amino acid sequence shown in SEQ ID No. 4 of the Sequence Listing, which includes substitution, deletion, insertion, addition, or inversion of one or plural amino acids and has cysteine protease inhibitory activity. "Substitution, deletion, insertion, addition, or inversion of one or plural amino acids" may be set arbitrary as long as it does not influence steric interaction between the protein or the peptide having amino acid sequence of the above-mentioned amino acid numbers and cysteine protease, and does not impair cysteine protease activity. For example, "plural" refers to 2 to 5 amino acids, preferably 2 or 3 amino acid, more preferably 2 amino acids. It varies with a position or a kind of an amino acid residue in the protein conformation of amino acid numbers 666 to 682 of the amino acid sequence shown in SEQ ID No. 3 of the Sequence Listing, or amino acid numbers 672 to 688 of the amino acid sequence shown in SEQ ID No. 4 of the Sequence Listing. Substitution in the amino acid of amino acid numbers 673 to 677 of the amino acid sequence shown in SEQ ID No. 3 of the Sequence Listing or in the amino acid of amino acid numbers 679 to 683 of the amino acid sequence shown in SEQ ID No. 4 of the Sequence Listing is preferably a substitution which does not change a kind of amino acid. Namely, it is preferable that the replacing amino acids in the above-mentioned sequence are the amino acid having the similar character in the structural classification as the replaced amino acid. Specifically, replacement by aspartic acid, glutamic acid, asparagine or glutamine are preferable when the amino acid in the above-mentioned sequence is acidic amino acid or amide of acidic amino acid, replacement by lysine, histidine or arginine are preferable in the case of basic amino acid, replacement by phenylalanine, tyrosine or tryptophan is preferable in the case of aromatic amino acid, replacement by glycine, alanine, valine, leucine, isoleucine, serine or threonine is preferable in the case of aliphatic amino acid or oxyamino acid, further, in the case of amino acid other than the above-mentioned amino acids (cysteine, methionine, proline etc.), it is preferable that replacement is performed arbitrary as long as it does not impair cysteine protease activity. Further, substitution, deletion and the like of one or plural amino acids may be included in amino acids except the amino acids of amino acid numbers 666 to 682 of the amino acid sequence shown in SEQ ID No. 3 of the Sequence Listing, or amino acids except the amino acids of amino acid numbers 672 to 688 of the amino acid sequence shown in SEQ ID No. 4 of the Sequence Listing. In this case, for example, the term plural refers to 2 to 10 amino acids, preferably 2 to 5 amino acids, more preferably 2 or 3 amino acids, though varying with a position or a kind of an amino acid residue in protein conformation.

Moreover, examples of transferrin or a partial peptide of transferrin which can be used for the present invention include a protein or a peptide which has homology not less than 80%, preferably not less than 90%, more preferably not less than 95% in an amino acid sequence with a protein or a peptide having amino acid sequence of at least amino acid numbers 666 to 682 of the amino acid sequence shown in SEQ ID No. 3 of the Sequence Listing, or a protein or peptide having amino acid sequence of at least amino acid numbers 672 to 688 of the amino acid sequence shown in SEQ ID No. 4 of the Sequence Listing, and which has cysteine protease inhibitory activity.

Nucleotide sequence which codes for a protein or a peptide substantially identical with the transferrin protein or partial peptide of transferrin as described above can be obtained, for example, by modifying the nucleotide sequence with site-directed mutagenesis in such a manner that an amino acid residue located at a specific site includes substitution, deletion, insertion, addition or inversion. The modified nucleotidesequence may be obtained by means of conventional mutagenesis treatment.

Nucleotide sequence which codes for protein or a peptide substantially identical with transferrin or partial peptide of transferrin may be obtained by expressing a nucleotide sequence including mutation in appropriate cells and examining cysteine protease inhibitory activity by a method of determining the cysteine protease inhibitory activity described in Test Examples or Examples of the present invention.

In the protease inhibitor of the present invention, lactoferrin, a partial peptide of lactoferrin, or transferrin, a partial peptide of transferrin may be used alone, or two or more thereof may be usedtogether. Moreover, one type of a partial peptide of lactoferrin may be used alone, or plural types thereof may be used in combination. Further, one type of a partial peptide of transferrin may be used alone, or plural types thereof may be used in combination.

Lactoferrin, a partial peptide of lactoferrin, or transferrin which can be used for the present invention has inhibitory activity against cysteine proteases such as cathepsin B, L, S and papain. The cysteine protease inhibitory activity can be determined in accordance with the methods of Barrett et al. (Methods in Enzymology, Vol. 80, pp. 535-561, 1981). The method of determination will be described in detail in Test Examples and Examples of the present invention.

The cysteine protease inhibitor of the present invention can be produced by using lactoferrin, a partial peptide of lactoferrin or transferrin, and combining them with a pharmaceutical acceptable carrier. A form of administration unit of the pharmaceutical preparation of the present invention is not particularly limited, and may be appropriately selected in accordance with therapeutic purposes. Specifically, examples of the formof administration unit include a tablet, a pill, powder, liquid, a suspension, an emulsion, granules, capsule, syrup, a suppository, injectable solution, an ointment, a patch, eye-drop and collunarium. Additives such as a vehicle, a bonding agent, a disintegrator, a lubricant, a stabilizer, a flavoring agent, diluent, surfactant and a solution for injection, which are commonly used for pharmaceuticals as a pharmaceutical carrier, may be used upon preparation.

The amount of lactoferrin, a partial peptide of lactoferrin or transferrin contained in the pharmaceutical of the present invention is not particularly limited and may be appropriately selected. For example, it may be usually set to 0.005 to 60% by mass, preferably 0.05 to 50% by mass in the preparation.

Diseases associated with cysteine protease can be treated by oral or parenteral administration of the cysteine protease inhibitor of the present invention to a subject. The term "subject" used herein may be either human beings or mammals other than human.

A method of administering the pharmaceutical of the present invention is not particularly limited, and can be determined according to a form of the pharmaceutical, a subject's age or sex, and other conditions including a degree of the subject's symptom. A dosage of an active ingredient of the pharmaceutical of the present invention can be appropriately set based on a dose regimen, a subject' s age or sex, a degree of a disease, and other conditions. Usually, the amount of lactoferrin, partial peptide of lactoferrin or transferrin as active ingredients may be set based on the amount ranging from 0.1 to 1,200 mg/kg/day, preferably 10 to 500 mg/kg/day, and the pharmaceutical may be administered once or plural times per day.

The cysteine protease inhibitor of the present invention is useful as preventive or therapeutic agents for diseases associated with cysteine protease, such as, allergy, muscular dystrophy, myocardial infarct, apoplexy, Alzheimer's disease, multiple sclerosis, cataract, osteoporosis, malignant hypercalcemia, enlarged prostate,breast cancer,prostate cancer,and periodontitis, or as an inhibitor of cancer cell growth or metastasis, or as a growth inhibitor for bacteria (Staphylococcus aureus V8, etc.) or viruses (poliovirus, herpesvirus, coronavirus, AIDS virus, etc.). The cysteine protease inhibitor of the present invention may be used alone, or used in combination with known preventive or therapeutic agents for the above-mentioned diseases or known growth inhibitor of the bacteria or viruses. Such combination can enhance preventive and therapeutic effects against the diseases, or a growth inhibitory effect against the bacteria or viruses. The known preventive and therapeutic agents for the above-mentioned diseases, or known growth inhibitor for the bacteria or viruses to be combined may be contained in the inhibitor of the present invention as an active ingredient, or may be commercialized as another agent and combined upon use without being contained in the inhibitor of the present invention.

The food and drink composition of the present invention may be produced by adding lactoferrin, partial peptide of lactoferrin or transferrin to raw materials for food or drink, and it can be orally ingested. As the raw materials, those employed in drink or food may be generally used. The food and drink composition of the present invention may be prepared in a similar way as usual food and drink composition except that the cysteine protease inhibitor is added. Examples of the forms of the food and drink composition include drinks such as cold drink, carbonated drink, nutrition drink, fruit drink and lactobacillus beverage (including concentrated stock solution and adjustment powder of those drinks); ice sweets such as an ice cream, sherbet and shaved ice; confectionery such as candy, chewing gum, gum, chocolate, confectionery pill, snack food, biscuit, jelly, jam, cream and baked confectionery; milk products such as processed milk, milk beverage, fermented milk and butter; bread; enteral nutrition formula, liquid formula, infant formula and sport drink; and other functional food.

The amount of lactoferrin, partial peptide of lactoferrin or transferrin to be added in the food and drink composition of the present invention is properly set according to the form of the food and drink composition, and normally they may be added in an amount of 0.005 to 60% by mass, preferably 0.05 to 50% by mass in the food or drink.

The feed composition of the present invention can be produced by adding lactoferrin, a partial peptide of lactoferrin or transferrin to feed, and orally administered to general mammals, livestock, pisciculture, farmed fish, and pet animals. Examples of the forms of feed composition include pet foods, livestock feed, and pisciculture feed, and the feed composition of the present invention can be produced by formulating lactoferrin, a partial peptide of lactoferrin or transferrin with grains, lees, rice bran, fish flour, bone manure, oils and fats, skim milk, whey, mineral feed, yeast and the like.

The amount of lactoferrin, a partial peptide of lactoferrin, or transferrin to be added in the feed composition of the present invention is properly set according to the form of the feed composition, and normally they may be added in an amount of 0.005 to 60% by mass, preferably 0.05 to 50% by mass in the feed composition.

The food and drink composition or the feed composition of the present invention may be a food and drink composition or feed composition having indication of its efficacy as preventive or treatment for the diseases shown below. That is, it can be indicated as a preventive or treatment for diseases associated with cysteine protease, such as osteoporosis, malignant hypercalcemia.

The term "indicated" used herein means informing the users of the above-mentioned efficacy, and includes, for example, indicating the above-mentioned efficacy on commercial products of the food and drink composition or the feed composition of the present invention or on packages or advertisement thereof, and transferrin, turning over and displaying the substances having such indication. In particular, an embodiment in which it is indicated as a food for specified health uses [refer to Article 12 (1), 5 of the regulation of Health Enforcement Law (April 30, 2003, Ordinance No. 86 of the Japanese Ministry of Health, Labor and Welfare)] is preferable.

Hereinafter, the present invention will be described in detail by showing Test Examples.

### [Test Example 1]

The present test was carried out to detect cysteine protease inhibitory substance in milk.

### (1) Detection method

The inventor used a technique "reverse zymography" as a method of detecting protease inhibitory substance and detected a protease inhibitory substance located on the gel of SDS-polyacrylamide gel electrophoresis. The reverse zymography is based on a technique opposite of the normal zymography, and the basic principle of the reverse zymography is as follows. That is, a sample containing protease inhibitory substance is applied onto SDS-polyacrylamide gel containing gelatin, and electrophoresis is performed, followed by soaking the gel in a protease solution to degrade protein in the gel. Protease activity is inhibited on a portion where an inhibitory substance is present and gelatin of the portion avoids being degraded by protease and is stained with staining solution, which enables detection of inhibitory substance.

### (2) Test method

A method of the reverse zymography in the present invention is as follows.

Using total milk protein and bovine lactoferrin (manufactured by Morinaga Milk Industry Co., Ltd.) as samples, electrophoresis (hereinafter, SDS-polyacrylamide gel electrophoresis is sometimes abbreviated to SDS-PAGE) was conducted with 12.5% SDS-polyacrylamide gel containing 0.1% of gelatin. After electrophoresis, the gel was washed by immersing it in a 2.5%Triton X-100 solution for 45 minutes, and further washed by repeating three times the operation of immersing the gel in distilled water for 45 minutes. Then, the gel was immersed in 100 ml of a 0.025 M acetic acid buffer solution (pH 5.5) containing 1 mg of papain (31units/ml), and gelatin was digested by keeping in the solution at 37 °C for 10 hours. The gel was washed with distilled water, stained with a staining solution (0.025% Coomassie brilliant blue (CBB) R-250, 40% methanol, 7% acetic acid aqueous solution) for one hour, and then destained with a destaining solution (40% methanol, 10% acetic acid aqueous solution).

Aside from this, as a control test, the reverse zymography was conducted with a 12.5% SDS-polyacrylamide gel not containing gelatin in the same way as above. Further, typical 12.5% SDS-PAGE (CBB staining) was also performed.

### (3) Test results

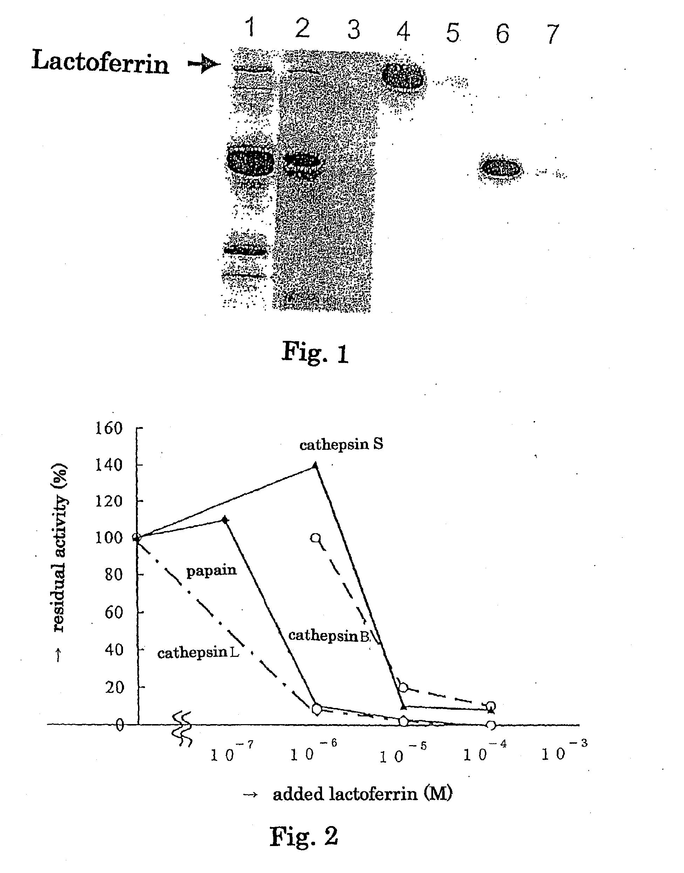

The results of the present test are as shown in Fig. 1. Fig. 1 shows the pattern of the reverse zymography. Lane 1 in Fig. 1 shows a typical SDS-PAGE pattern of the total protein in milk, lane 2 shows a pattern of the reverse zymography of the total protein in milk, lane 3 shows a pattern of the reverse zymography (control), in which the gel does not contain gelatin, of the total protein in milk, lane 4 shows a pattern of the reverse zymography of bovine lactoferrin, and lane 5 shows a pattern of the reverse zymography (control), in which the gel does not contain gelatin, of bovine lactoferrin, respectively. Arrows in the figure show a position of migration of bovine lactoferrin (78kDa in molecular weight) in SDS-PAGE. On the other hand, lanes 6 and 7 have no direct relations with the present test examples.

As is apparent from Fig. 1, a positive band of the reverse zymography was found in the position almost identical to the position of migration of bovine lactoferrin (78kDa) in lane 2. This ensured the presence of a substance having cysteine protease inhibitory activity in milk. In addition, a positive band was found in lane 4 which shows the reverse zymography using papain in which bovine lactoferrin was migrated.

The above-described results suggested that lactoferrin derived from bovine has cysteine protease-inhibitory activity.

### [Test Example 2]

The present test was carried out to determine the N-terminal amino acid sequence of a band of 78 kDa which was suggested to have cysteine protease inhibitory activity in Test Example 1.

### (1) Test method

SDS-PAGE was carried out with total protein sample in milk used in Test Example 1, and proteins were transferred to polyvinylidene difluoride (PVDF) membrane. The PVDF membrane was stained with CBB, and then the stained band migrating in the vicinity of 78 kDa was cut out. The N-termini amino acid sequence of this band was determined with a G1005A Protein Sequencing System, manufactured by Hewlett-Packard Company.

### (2)Test result

As a result of determining the amino acid sequence of the stained band of 78 kDa which was derived from milk, the N-terminal amino acid sequence of the 78 kDa stained band was found to be completely consistent with that of bovine lactoferrin as described in SEQ ID No.2 of the Sequence Listing. Accordingly, both results of the present test and Test Example 1 revealed that bovine lactoferrin has cysteine protease inhibitory activity.

### [Test Example 3]

The present test was carried out to determine an inhibitory activity of bovine lactoferrin to cysteine proteases.

### (1) Test method

Inhibitory activities against cysteine proteases including papain, cathepsin B, cathepsin L and cathepsin S (these proteases are available from Wako Pure Chemical Industries, Ltd.) were determined using commercially available bovine lactoferrin (manufactured by Morinaga Milk Industry Co., Ltd.) as a test sample. The inhibitory activity was measured with reference to the method by Barrett et al. ("Methods in Enzymology," Vol. 80, p 535-561, 1981) as described below. Namely, Z-Phe-Arg-MCA (final concentration of 20 mM: manufactured by Peptide Institute, Inc.) was added as a substrate to 0.1 M acetic acid buffer solution (pH 5.5) in which sample was dissolved at each concentration. Then, cysteine protease (a protease selected from papain, cathepsin B, cathepsin L and cathepsin S in the present test: manufactured by Wako Pure Chemical Industries, Ltd.) solution (final concentration: 15 units/ml) was added, the whole solution was mixed and the reaction was carried out at 37°C for 10 minutes. Subsequently, fluorescence intensity (excitation wavelength: 370 nm, emission wavelength: 460 nm) of AMC (7-Amino-4-Methyl-Coumarin) released from the digested substrate was measured with a fluorescence spectrometer (manufactured by Hitachi, Ltd.).

### (2) Test result

The results of the present test are as shown in Table 1. Table 1 shows cysteine protease-inhibitory activity of bovine lactoferrin on papain, cathepsin B, cathepsin L and cathepsin S. The results revealed that cysteine protease activities of papain and cathepsin L were completely inhibited by 10⁻⁶ M of bovine lactoferrin, and that cysteine protease activities of cathepsin B and cathepsin S were inhibited more than 70% by 10⁻⁵ M of bovine lactoferrin and completely inhibited by 10⁻⁴ M of bovine lactoferrin. Accordingly, bovine lactoferrin was found to have cysteine protease-inhibitory activity against papain, cathepsin B, cathepsin L and cathepsin S.

**[Table 1]**

| inhibitor | cysteine protease | inhibition rate at each concentration(%) | | | | |
|---|---|---|---|---|---|---|
| | | 10⁻⁸M | 10⁻⁷M | 10⁻⁶M | 10⁻⁵M | 10⁻⁴M |
| bovine lactoferrin | papain | 0 | 40 | 100 | 100 | 100 |
| | cathepsin L | 0 | 20 | 100 | 100 | 100 |
| | cathepsin B | 0 | 0 | 10 | 70 | 100 |
| | cathepsin S | 0 | 0 | 15 | 80 | 100 |

### [Test Example 4]

The present test was carried out to determine an inhibitory activity of human lactoferrin to cysteine proteases.

### (1) Test method

Inhibitory activities against cysteine proteases including papain, cathepsin B, cathepsin L and cathepsin S (these proteases are available from Wako Pure Chemical Industries, Ltd.) were determined using commercially available human lactoferrin (manufactured by Morinaga Milk Industry Co., Ltd.) as a test sample, according to the same method as that of determining cysteine protease-inhibitory activity described in the Test Example 3.

### (2) Test results

The results of the present test are as shown in Fig. 2. Fig. 2 shows cysteine protease-inhibitory activity of human lactoferrin to papain, cathepsin B, cathepsin L and cathepsin S. The results revealed that cysteine protease activities of papain and cathepsin L were completely inhibited by 10⁻⁶ M of human lactoferrin, and that cysteine protease activities of cathepsin B and cathepsin S were inhibited more than 80% by 10⁻⁵ M of human lactoferrin and almost completely inhibited by 10⁻⁴ M of human lactoferrin. Accordingly, human lactoferrin was found to have cysteine protease-inhibitory activity against papain, cathepsin B, cathepsin L and cathepsin S.

### [Test Example 5]

The present test was carried out to determine an inhibitory activity of a partial peptide of lactoferrin to cysteine proteases.

### (1) Test method

Inhibitory activities against cysteine proteases including papain, cathepsin B and cathepsin L (these proteases are available from Wako Pure Chemical Industries, Ltd.) were determined using a peptide having an amino acid sequence of amino acid numbers 679-695 of the amino acid sequence of SEQ ID No.1 in the Sequence Listing (hereinafter, described as human lactoferrin peptide Y679-K695, Figure 3) as a test sample, according to the same method as the method of determining cysteine protease-inhibitory activity described in the Test Example 3.

### (2) Test results

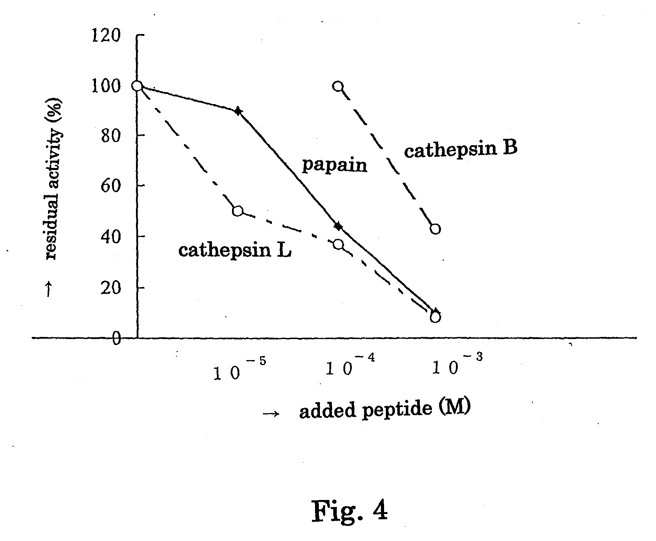

The results of the present test are as shown in Fig. 4. Fig. 4 shows cysteine protease-inhibitory activity of human lactoferrin peptide Y679-K695 on papain, cathepsin B and cathepsin L. The results revealed that cysteine protease activities of papain and cathepsin L were inhibited more than 50% by 10⁻⁴ M of human lactoferrin peptide Y679-K695 and almost completely inhibited by 10⁻³ M of human lactoferrin peptide Y679-K695, and that cysteine protease activity of cathepsin B was inhibited nearly 60% by 10⁻³ M of human lactoferrin peptide Y679-K695. Accordingly, human lactoferrin peptide Y679-K695 was found to have cysteine protease-inhibitory activity against papain, cathepsin B and cathepsin L. Further, similar test for determining cysteine protease-inhibitory activity was performed using a peptide having an amino acid sequence of amino acid numbers 676-692 of the amino acid sequence of SEQ ID No.2 in the Sequence Listing (hereinafter, described as bovine lactoferrin peptide Y676-K692, Figure 3), and it was found that the bovine lactoferrin peptide Y676-K692 has an activity almost equal to the human lactoferrin peptide Y679-K695.

### [Test Example 6]

The present test was carried out to determine an inhibitory activity of transferrin to cysteine proteases.

### (1) Test method

Inhibitory activities against cysteine proteases including papain, cathepsin B, and cathepsin L (these proteases are available from Wako Pure Chemical Industries, Ltd.) were determined using commercially available bovine transferrin (manufactured by Nihon Pharmaceutical Co., Ltd.) as a test sample, according to the same method as that of determining cysteine protease-inhibitory activity described in the Test Example 3.

### (2) Test results

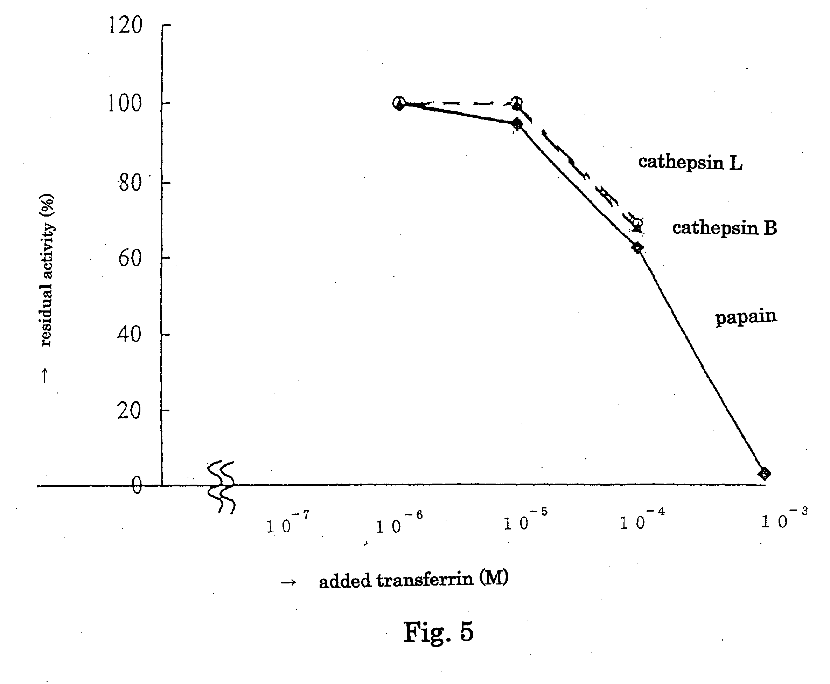

The results of the present test are as shown in Fig. 5. Fig. 5 shows cysteine protease-inhibitory activity of transferrin to papain, cathepsin B and cathepsin L. The results revealed that cysteine protease activity of papain was inhibited 40% by 10⁻⁴ M of transferrin and completely inhibited by 10⁻³ M of transferrin, and that cysteine protease activities of cathepsin B and cathepsin L were inhibited more than 30% by 10⁻⁴ M of transferrin. Accordingly, bovine transferrin was found to have cysteine protease-inhibitory activity against papain, cathepsin B and cathepsin L.

### Examples

Next, the present invention will be more specifically described by showing examples. However, the present invention is not limited to the following examples.

### [Example 1]

A peptide having an amino acid sequence of amino acid numbers 679-695 of the amino acid sequence of SEQ ID No.1 in the Sequence Listing (Figure 3, human lactoferrin peptide Y679-K695) was produced according to the following procedure.

The above-mentioned peptide of the present invention was produced by synthesizing it with an automatic peptide synthesizer (manufactured by Applied Biosystems Co., Ltd., Model 433A).

Fmoc-group, an amino protective group of HMP resin (manufactured by Applied Biosystems Co., Ltd.) which is a solidified resin for peptide synthesis, was cleaved with N-metylpyrrolidone (manufactured by Applied Biosystems Co., Ltd., hereinafter, abbreviated to NMP) containing 20% of piperidine, and the resin was washed with NMP. Then, Fmoc-threonine [specifically, Fmoc-amino acid (manufactured by Applied Biosystems Co., Ltd.) corresponding to the C-terminal amino acid of the peptide to be synthesized] was condensed to the resin using FastMoc (registered trademark) reagent kit (Applied Biosystems Co., Ltd.), and the resin was washed with NMP. Next, the Fmoc group was cleaved again, and Fmoc-alanine corresponding to the second amino acid from the C-terminus was condensed, followed by washing the resin. Protective peptide resin was prepared by further repeating condensation of Fmoc-amino acid and washing, and crude peptide was recovered from the resin.

Peptides were purified from the crude peptides using high performance liquid chromatography (hereinafter, abbreviated to HPLC). C18-ODS (manufactured by Merck & Co, Inc. , Lichrospher 100), a reverse phase column, was used as a column. The obtained purified peptides were analyzed by HPLC to further confirm that the purified product is a single peptide. The amino acid sequence of the purified peptide was determined by use of a gas-phase automatic amino acid sequencer (manufactured by Applied Biosystems Co., Ltd., Model 473A), and it was found to have amino acid sequence of amino acid numbers 679 to 695 in the SEQ ID No. 1.

According to the similar method, a peptide having amino acid sequence of amino acid numbers 676 to 692 in the amino acid sequence described in SEQ ID No. 2 (Figure 3, bovine lactoferrin peptide Y676-K692) was produced.

### [Example 2]

### (Preparation of a tablet in which lactoferrin is formulated)

A tablet of a cysteine protease inhibitor having the following compositions was produced according to the following procedures.
Bovine lactoferrin (manufactured by Morinaga Milk Industry Co., Ltd.) 40.0(%)
Lactose (manufactured by Morinaga Milk Industry Co., Ltd.) 18.5 Cornstarch (manufactured by Nisshin Flour Milling Co., Ltd.) 30.7 Magnesium stearate (manufactured by Taihei Chemical Industrial Co. , Ltd.) 1.4
Carboxymethyl cellulose calcium (manufactured by Gotoku Chemical Co., Ltd.) 9.4

A mixture of bovine lactoferrin, lactose, corn starch and carboxymethyl cellulose calcium was kneaded uniformly while sterile purified water was appropriately added, and the kneaded product was dried at 50°C for three hours. Magnesium stearate was then added to the obtained dried product and mixed, followed by compression according to a conventional procedure, thereby a tablet was obtained.

### [Example 3]

### (Preparation of encapsulated lactoferrin)

600 g of lactose (manufactured by Wako Pure Chemical Industries, Ltd.), 400 g of corn starch (manufactured by Nisshin Flour Milling Co., Ltd.), 400 g of crystalline cellulose (manufactured by Wako Pure Chemical Industries, Ltd.) and 600 g of bovine lactoferrin (manufactured by Morinaga Milk Industry Co., Ltd.) were sieved with 50 mesh sieve (manufactured by Yamato Scientific Co., Ltd.), and put into a polyethylene bag with a thickness of 0.5 mm to allow to mix upside down. The obtained powder was packed into a capsule (manufactured by Shionogi Qualicaps, Inc., gelatin capsule No. 1, Op Yellow No. 6 Body, 75 mg of empty weight) in a content of 275 mg using a fully automatic capsule filling machine (manufactured by Cesere Pedini, press type), thereby 7, 000 capsules each containing 82 mg of bovine lactoferrin were obtained.

### [Example 4]

### (Preparation of drink in which bovine lactoferrin is added)

90 g of skim milk (manufactured by Morinaga Milk Industry Co. , Ltd.) were dissolved in 800 ml of hot water at a temperature of 50°C, and 30 g of sugar (manufactured by Nissin Sugar Manufacturing Co., Ltd.), 14 g of instant coffee powder (manufactured by Nestle), 2 g of caramel (manufactured by Showa Kako Co., Ltd.) and 0.01 g of coffee flavor (manufactured by San-Ei Gen F.F.I. Inc.) were sequentially added and dissolved in the solution while stirring. Then, the mixture was cooled to 10°C, and 1 g of bovine lactoferrin (manufactured by Sigma Co., Ltd.) was added, thereby milk beverage containing about 0.1% of bovine lactoferrin and having cysteine protease-inhibitory activity was prepared.

### [Example 5]

### (Preparation of powder of enteral nutrition formula in which bovine lactoferrin is added)

10.8 kg of enzyme degradation product of whey protein (manufactured by Morinaga Milk Industry Co., Ltd.), 36 kg of dextrin (manufactured by Showa Sangyo Co., Ltd.), and a small amount of water-soluble vitamin and minerals were dissolved in 200 kg of water to prepare an aqueous phase in a tank. Aside from this, 3 kg of soybean salad oil (manufactured by Taiyo Yushi Co., Ltd.), 8.5 kg of palm oil (manufactured by Taiyo Yushi Co., Ltd.), 2.5 kg of safflower oil (manufactured by Taiyo Yushi Co., Ltd.), 0.2 kg of lecithin (manufactured by Ajinomoto Co., Inc.), 0.2 kg of fatty acid monoglyceride (manufactured by Kao Corporation) and a small amount of fat-soluble vitamin were mixed and dissolved to prepare an oil phase. The oil phase was added to the aqueous phase in the tank and the whole was mixed by stirring, followed by heating to 70°C and homogenizing by a homogenizer under a pressure of 14.7 MPa. Next, the resultant was sterilized at 90°C for 10 minutes, concentrated and spray-dried, thereby approximately 59 kg of powder of intermediate product was obtained. Then, 6.8 kg of sucrose (manufactured by Hokuren-Federation of Agriculture Cooperatives), 167 g of amino acid mixture powder (manufactured by Ajinomoto Co., Inc.) and 60 g of bovine lactoferrin (manufactured by Sigma Co., Ltd.) were added to 50 kg of the powder of the intermediate product, and uniformly mixed, thereby approximately 57 kg of enteral nutrition formula containing bovine lactoferrin and having a cysteine protease-inhibitory activity was obtained.

### Industrial Applicability

As described in detail above, the present invention relates to a cysteine protease inhibitor comprising one or more of lactoferrin, a partial peptide of lactoferrin, and transferrin, as an active ingredient. Effects exerted by the present invention are as follows:
(1) Excellent in safety and capable of being administrated or ingested daily in a long term because it is made of a protein available as food material or a partial peptide thereof.
(2) Having a wide spectrum of inhibitory activity on various cysteine proteases.
(3) Usable as preventive or therapeutic agents for diseases associated with cysteine protease.
(4) Applicable to production of functional food including food for specified health uses and nutritional supplementary food.
(5) Usable as an agent for adjusting physical properties of food in the field of food processing.

## Claims

1. A cysteine protease inhibitor comprising one or more of lactoferrin, a peptide fragment of lactoferrin, and transferrin, as an active ingredient.

2. The cysteine protease inhibitor according to claim 1, wherein lactoferrin and/or transferrin are one or more of metal-saturated form, partially metal-saturated form and apo form.

3. The cysteine protease inhibitor according to claim 1 or 2, wherein lactoferrin and/or transferrin are one or more of the proteins or peptides as shown in the following (a) to (d);
(a) a protein or peptide having amino acid sequence of at least amino acid numbers 679 to 695 of the amino acid sequence shown in SEQ ID No. 1 of the Sequence Listing,
(b) a protein or peptide having amino acid sequence of at least amino acid numbers 679 to 695 of the amino acid sequence shown in the SEQ ID No. 1 of the Sequence Listing, including substitution, deletion, addition or inversion of one or plural amino acids, and having cysteine protease inhibitory activity,
(c) a protein or peptide having amino acid sequence of at least amino acid numbers 676 to 692 of the amino acid sequence shown in SEQ ID No. 2 of the Sequence Listing, and
(d) a protein or peptide having amino acid sequence of at least amino acid numbers 676 to 692 of the amino acid sequence shown in the SEQ ID No. 2 of the Sequence Listing, including substitution, deletion, addition or inversion of one or plural amino acids, and having cysteine protease inhibitory activity.

4. The cysteine protease inhibitor according to any one of claims 1 to 3, which is a preventive or therapeutic agent for a disease associated with cysteine protease.

5. The cysteine protease inhibitor according to claim 4, wherein the disease associated with cysteine protease is osteoporosis or malignant hypercalcemia.

6. A food and drink composition or feed composition, which is produced by adding the cysteine protease inhibitor according to any one of claims 1 to 5.

7. A method for treating a disease associated with cysteine protease, wherein the cysteine protease inhibitor according to any one of claims 1 to 6 is administered to a subject.

8. A use of one or more of lactoferrin, a peptide fragment of lactoferrin, and transferrin, in manufacture of a cysteine protease inhibitor.

9. The use according to claim 8, wherein lactoferrin and/or transferrin are one or more of metal-saturated form, partially metal-saturated form and apo form.

10. The use according to claim 8 or 9, wherein lactoferrin and/or transferrin are one or more of the proteins or peptides as shown in the following (a) to (d);
(a) a protein or peptide having amino acid sequence of at least amino acid numbers 679 to 695 of the amino acid sequence shown in SEQ ID No. 1 of the Sequence Listing,
(b) a protein or peptide having amino acid sequence of at least amino acid numbers 679 to 695 of the amino acid sequence shown in the SEQ ID No. 1 of the Sequence Listing, including substitution, deletion, addition or inversion of one or plural amino acids, and having cysteine protease inhibitory activity,
(c) a protein or peptide having amino acid sequence of at least amino acid numbers 676 to 692 of the amino acid sequence shown in SEQ ID No. 2 of the Sequence Listing, and
(d) a protein or peptide having amino acid sequence of at least amino acid numbers 676 to 692 of the amino acid sequence shown in the SEQ ID No. 2 of the Sequence Listing, including substitution, deletion, addition or inversion of one or plural amino acids, and having cysteine protease inhibitory activity.

11. The use according to any one of claims 8 to 10, wherein the cysteine protease inhibitor is a preventive and therapeutic agent for a disease associated with cysteine protease.

12. The use according to claim 11, wherein the disease associated with cysteine protease is osteoporosis or malignant hypercalcemia.


SEQUENCE LISTING

<110> Morinaga Milk Industry Co., Ltd.

<120> Protease Inhibitor

<130> FP1470P1610

<150> JP 2002/347802
<151> 2002-11-29

<160> 4

<170> PatentIn Ver. 2.1

<210> 1
<211> 711
<212> PRT
<213> Homo Sapiens

<220>
<221> sig_peptide
<222> (1)…(19)
<223> Lactoferrin

<400> 1

<210> 2
<211> 708
<212> PRT
<213> Bos taurus

<220>
<221> sig_peptide
<222> (1)…(19)
<223> Lactoferrin

<400> 2
Met Lys Leu Phe Val Pro Ala Leu Leu Ser Leu Gly Ala Leu Gly Leu
1               5                   10                  15
Cys Leu Ala Ala Pro Arg Lys Asn Val Arg Trp Cys Thr Ile Ser Gln
            20                  25                  30
Pro Glu Trp Phe Lys Cys Arg Arg Trp Gln Trp Arg Met Lys Lys Leu
        35                  40                  45
Gly Ala Pro Ser Ile Thr Cys Val Arg Arg Ala Phe Ala Leu Glu Cys
    50                  55                  60
Ile Arg Ala Ile Ala Glu Lys Lys Ala Asp Ala Val Thr Leu Asp Gly
65                  70                  75                  80
Gly Met Val Phe Glu Ala Gly Arg Asp Pro Tyr Lys Leu Arg Pro Val
                85                  90                  95
Ala Ala Glu Ile Tyr Gly Thr Lys Glu Ser Pro Gln Thr His Tyr Tyr
            100                 105                 110
Ala Val Ala Val Val Lys Lys Gly Ser Asn Phe Gln Leu Asp Gln Leu
        115                 120                 125

Gln Gly Arg Lys Ser Cys His Thr Gly Leu Gly Arg Ser Ala Gly Trp
    130                 135                 140
Ile Ile Pro Met Gly Ile Leu Arg Pro Tyr Leu Ser Trp Thr Glu Ser
145                 150                 155                 160
Leu Glu Pro Leu Gln Gly Ala Val Ala Lys Phe Phe Ser Ala Ser Cys
                165                 170                 175
Val Pro Cys Ile Asp Arg Gln Ala Tyr Pro Asn Leu Cys Gln Leu Cys
            180                 185                 190
Lys Gly Glu Gly Glu Asn Gln Cys Ala Cys Ser Ser Arg Glu Pro Tyr
        195                 200                 205
Phe Gly Tyr Ser Gly Ala Phe Lys Cys Leu Gln Asp Gly Ala Gly Asp
    210                 215                 220
Val Ala Phe Val Lys Glu Thr Thr Val Phe Glu Asn Leu Pro Glu Lys
225                 230                 235                 240
Ala Asp Arg Asp Gln Tyr Glu Leu Leu Cys Leu Asn Asn Ser Arg Ala
                245                 250                 255
Pro Val Asp Ala Phe Lys Glu Cys His Leu Ala Gln Val Pro Ser His
            260                 265                 270
Ala Val Val Ala Arg Ser Val Asp Gly Lys Glu Asp Leu Ile Trp Lys
        275                 280                 285
Leu Leu Ser Lys Ala Gln Glu Lys Phe Gly Lys Asn Lys Ser Arg Ser
    290                 295                 300
Phe Gln Leu Phe Gly Ser Pro Pro Gly Gln Arg Asp Leu Leu Phe Lys
305                 310                 315                 320
Asp Ser Ala Leu Gly Phe Leu Arg Ile Pro Ser Lys Val Asp Ser Ala
                325                 330                 335
Leu Tyr Leu Gly Ser Arg Tyr Leu Thr Thr Leu Lys Asn Leu Arg Glu
            340                 345                 350
Thr Ala Glu Glu Val Lys Ala Arg Tyr Thr Arg Val Val Trp Cys Ala
        355                 360                 365
Val Gly Pro Glu Glu Gln Lys Lys Cys Gln Gln Trp Ser Gln Gln Ser
    370                 375                 380
Gly Gln Asn Val Thr Cys Ala Thr Ala Ser Thr Thr Asp Asp Cys Ile
385                 390                 395                 400
Val Leu Val Leu Lys Gly Glu Ala Asp Ala Leu Asn Leu Asp Gly Gly
                405                 410                 415

Tyr Ile Tyr Thr Ala Gly Lys Cys Gly Leu Val Pro Val Leu Ala Glu
            420                 425                 430
Asn Arg Lys Ser Ser Lys His Ser Ser Leu Asp Cys Val Leu Arg Pro
        435                 440                 445
Thr Glu Gly Tyr Leu Ala Val Ala Val Val Lys Lys Ala Asn Glu Gly
    450                 455                 460
Leu Thr Trp Asn Ser Leu Lys Asp Lys Lys Ser Cys His Thr Ala Val
465                 470                 475                 480
Asp Arg Thr Ala Gly Trp Asn Ile Pro Met Gly Leu Ile Val Asn Gln
                485                 490                 495
Thr Gly Ser Cys Ala Phe Asp Glu Phe Phe Ser Gln Ser Cys Ala Pro
            500                 505                 510
Gly Ala Asp Pro Lys Ser Arg Leu Cys Ala Leu Cys Ala Gly Asp Asp
        515                 520                 525
Gln Gly Leu Asp Lys Cys Val Pro Asn Ser Lys Glu Lys Tyr Tyr Gly
    530                 535                 540
Tyr Thr Gly Ala Phe Arg Cys Leu Ala Glu Asp Val Gly Asp Val Ala
545                 550                 555                 560
Phe Val Lys Asn Asp Thr Val Trp Glu Asn Thr Asn Gly Glu Ser Thr
                565                 570                 575
Ala Asp Trp Ala Lys Asn Leu Asn Arg Glu Asp Phe Arg Leu Leu Cys
            580                 585                 590
Leu Asp Gly Thr Arg Lys Pro Val Thr Glu Ala Gln Ser Cys His Leu
        595                 600                 605
Ala Val Ala Pro Asn His Ala Val Val Ser Arg Ser Asp Arg Ala Ala
    610                 615                 620
His Val Lys Gln Val Leu Leu His Gln Gln Ala Leu Phe Gly Lys Asn
625                 630                 635                 640
Gly Lys Asn Cys Pro Asp Lys Phe Cys Leu Phe Lys Ser Glu Thr Lys
                645                 650                 655
Asn Leu Leu Phe Asn Asp Asn Thr Glu Cys Leu Ala Lys Leu Gly Gly
            660                 665                 670
Arg Pro Thr Tyr Glu Glu Tyr Leu Gly Thr Glu Tyr Val Thr Ala Ile
        675                 680                 685
Ala Asn Leu Lys Lys Cys Ser Thr Ser Pro Leu Leu Glu Ala Cys Ala
    690                 695                 700

Phe Leu Thr Arg
705

<210> 3
<211> 698
<212> PRT
<213> Homo Sapiens

<220>
<221> sig_peptide
<222> (1)…(19)
<223> Transferrin

<400> 3
Met Arg Leu Ala Val Gly Ala Leu Leu Val Cys Ala Val Leu Gly Leu
1               5                   10                  15
Cys Leu Ala Val Pro Asp Lys Thr Val Arg Trp Cys Ala Val Ser Glu
            20                  25                  30
His Glu Ala Thr Lys Cys Gln Ser Phe Arg Asp His Met Lys Ser Val
        35                  40                  45
Ile Pro Ser Asp Gly Pro Ser Val Ala Cys Val Lys Lys Ala Ser Tyr
    50                  55                  60
Leu Asp Cys Ile Arg Ala Ile Ala Ala Asn Glu Ala Asp Ala Val Thr
65                  70                  75                  80
Leu Asp Ala Gly Leu Val Tyr Asp Ala Tyr Leu Ala Pro Asn Asn Leu
                85                  90                  95
Lys Pro Val Val Ala Glu Phe Tyr Gly Ser Lys Glu Asp Pro Gln Thr
            100                 105                 110
Phe Tyr Tyr Ala Val Ala Val Val Lys Lys Asp Ser Gly Phe Gln Met
        115                 120                 125
Asn Gln Leu Arg Gly Lys Lys Ser Cys His Thr Gly Leu Gly Arg Ser
    130                 135                 140
Ala Gly Trp Asn Ile Pro Ile Gly Leu Leu Tyr Cys Asp Leu Pro Glu
145                 150                 155                 160
Pro Arg Lys Pro Leu Glu Lys Ala Val Ala Asn Phe Phe Ser Gly Ser
                165                 170                 175

Cys Ala Pro Cys Ala Asp Gly Thr Asp Phe Pro Gln Leu Cys Gln Leu
            180                 185                 190
Cys Pro Gly Cys Gly Cys Ser Thr Leu Asn Gln Tyr Phe Gly Tyr Ser
        195                 200                 205
Gly Ala Phe Lys Cys Leu Lys Asp Gly Ala Gly Asp Val Ala Phe Val
    210                 215                 220
Lys His Ser Thr Ile Phe Glu Asn Leu Ala Asn Lys Ala Asp Arg Asp
225                 230                 235                 240
Gln Tyr Glu Leu Leu Cys Leu Asp Asn Thr Arg Lys Pro Val Asp Glu
                245                 250                 255
Tyr Lys Asp Cys His Leu Ala Gln Val Pro Ser His Thr Val Val Ala
            260                 265                 270
Arg Ser Met Gly Gly Lys Glu Asp Leu Ile Trp Glu Leu Leu Asn Gln
        275                 280                 285
Ala Gln Glu His Phe Gly Lys Asp Lys Ser Lys Glu Phe Gln Leu Phe
    290                 295                 300
Ser Ser Pro His Gly Lys Asp Leu Leu Phe Lys Asp Ser Ala His Gly
305                 310                 315                 320
Phe Leu Lys Val Pro Pro Arg Met Asp Ala Lys Met Tyr Leu Gly Tyr
                325                 330                 335
Glu Tyr Val Thr Ala Ile Arg Asn Leu Arg Glu Gly Thr Cys Pro Glu
            340                 345                 350
Ala Pro Thr Asp Glu Cys Lys Pro Val Lys Trp Cys Ala Leu Ser His
        355                 360                 365
His Glu Arg Leu Lys Cys Asp Glu Trp Ser Val Asn Ser Val Gly Lys
    370                 375                 380
Ile Glu Cys Val Ser Ala Glu Thr Thr Glu Asp Cys Ile Ala Lys Ile
385                 390                 395                 400
Met Asn Gly Glu Ala Asp Ala Met Ser Leu Asp Gly Gly Phe Val Tyr
                405                 410                 415
Ile Ala Gly Lys Cys Gly Leu Val Pro Val Leu Ala Glu Asn Tyr Asn
            420                 425                 430
Lys Ser Asp Asn Cys Glu Asp Thr Pro Glu Ala Gly Tyr Phe Ala Val
        435                 440                 445
Ala Val Val Lys Lys Ser Ala Ser Asp Leu Thr Trp Asp Asn Leu Lys
    450                 455                 460

Gly Lys Lys Ser Cys His Thr Ala Val Gly Arg Thr Ala Gly Trp Asn
465                 470                 475                 480
Ile Pro Met Gly Leu Leu Tyr Asn Lys Ile Asn His Cys Arg Phe Asp
                485                 490                 495
Glu Phe Phe Ser Glu Gly Cys Ala Pro Gly Ser Lys Lys Asp Ser Ser
            500                 505                 510
Leu Cys Lys Leu Cys Met Gly Ser Gly Leu Asn Leu Cys Glu Pro Asn
        515                 520                 525
Asn Lys Glu Gly Tyr Tyr Gly Tyr Thr Gly Ala Phe Arg Cys Leu Val
    530                 535                 540
Glu Lys Gly Asp Val Ala Phe Val Lys His Gln Thr Val Pro Gln Asn
545                 550                 555                 560
Thr Gly Gly Lys Asn Pro Asp Pro Trp Ala Lys Asn Leu Asn Glu Lys
                565                 570                 575
Asp Tyr Glu Leu Leu Cys Leu Asp Gly Thr Arg Lys Pro Val Glu Glu
            580                 585                 590
Tyr Ala Asn Cys His Leu Ala Arg Ala Pro Asn His Ala Val Val Thr
        595                 600                 605
Arg Lys Asp Lys Glu Ala Cys Val His Lys Ile Leu Arg Gln Gln Gln
    610                 615                 620
His Leu Phe Gly Ser Asn Val Thr Asp Cys Ser Gly Asn Phe Cys Leu
625                 630                 635                 640
Phe Arg Ser Glu Thr Lys Asp Leu Leu Phe Arg Asp Asp Thr Val Cys
                645                 650                 655
Leu Ala Lys Leu His Asp Arg Asn Thr Tyr Glu Lys Tyr Leu Gly Glu
            660                 665                 670
Glu Tyr Val Lys Ala Val Gly Asn Leu Arg Lys Cys Ser Thr Ser Ser
        675                 680                 685
Leu Leu Glu Ala Cys Thr Phe Arg Arg Pro
    690                 695

<210> 4
<211> 704
<212> PRT
<213> Bos taurus

<220>
<221> sig_peptide
<222> (1)···(19)
<223> Transferrin

<400> 4
Met Arg Pro Ala Val Arg Ala Leu Leu Ala Cys Ala Val Leu Gly Leu
1               5                   10                  15
Cys Leu Ala Asp Pro Glu Arg Thr Val Arg Trp Cys Thr Ile Ser Thr
            20                  25                  30
His Glu Ala Asn Lys Cys Ala Ser Phe Arg Glu Asn Val Leu Arg Ile
        35                  40                  45
Leu Glu Ser Gly Pro Phe Val Ser Cys Val Lys Lys Thr Ser His Met
    50                  55                  60
Asp Cys Ile Lys Ala Ile Ser Asn Asn Glu Ala Asp Ala Val Thr Leu
65                  70                  75                  80
Asp Gly Gly Leu Val Tyr Glu Ala Gly Leu Lys Pro Asn Asn Leu Lys
                85                  90                  95
Pro Val Val Ala Glu Phe His Gly Thr Lys Asp Asn Pro Gln Thr His
            100                 105                 110
Tyr Tyr Ala Val Ala Val Val Lys Lys Asp Thr Asp Phe Lys Leu Asn
        115                 120                 125
Glu Leu Arg Gly Lys Lys Ser Cys His Thr Gly Leu Gly Arg Ser Ala
    130                 135                 140
Gly Trp Asn Ile Pro Met Ala Lys Leu Tyr Lys Glu Leu Pro Asp Pro
145                 150                 155                 160
Gln Glu Ser Ile Gln Arg Ala Ala Ala Asn Phe Phe Ser Ala Ser Cys
                165                 170                 175
Val Pro Cys Ala Asp Gln Ser Ser Phe Pro Lys Leu Cys Gln Leu Cys
            180                 185                 190
Ala Gly Lys Gly Thr Asp Lys Cys Ala Cys Ser Asn His Glu Pro Tyr
        195                 200                 205
Phe Gly Tyr Ser Gly Ala Phe Lys Cys Leu Met Glu Gly Ala Gly Asp
    210                 215                 220
Val Ala Phe Val Lys His Ser Thr Val Phe Asp Asn Leu Pro Asn Pro
225                 230                 235                 240

Glu Asp Arg Lys Asn Tyr Glu Leu Leu Cys Gly Asp Asn Thr Arg Lys
                245                 250                 255
Ser Val Asp Asp Tyr Gln Glu Cys Tyr Leu Ala Met Val Pro Ser His
            260                 265                 270
Ala Val Val Ala Arg Thr Val Gly Gly Lys Glu Asp Val Ile Trp Glu
        275                 280                 285
Leu Leu Asn His Ala Gln Glu His Phe Gly Lys Asp Lys Pro Asp Asn
    290                 295                 300
Phe Gln Leu Phe Gln Ser Pro His Gly Lys Asp Leu Leu Phe Lys Asp
305                 310                 315                 320
Ser Ala Asp Gly Phe Leu Lys Ile Pro Ser Lys Met Asp Phe Glu Leu
                325                 330                 335
Tyr Leu Gly Tyr Glu Tyr Val Thr Ala Leu Gln Asn Leu Arg Glu Ser
            340                 345                 350
Lys Pro Pro Asp Ser Ser Lys Asp Glu Cys Met Val Lys Trp Cys Ala
        355                 360                 365
Ile Gly His Gln Glu Arg Thr Lys Cys Asp Arg Trp Ser Gly Phe Ser
    370                 375                 380
Gly Gly Ala Ile Glu Cys Glu Thr Ala Glu Asn Thr Glu Glu Cys Ile
385                 390                 395                 400
Ala Lys Ile Met Lys Gly Glu Ala Asp Ala Met Ser Leu Asp Gly Gly
                405                 410                 415
Tyr Leu Tyr Ile Ala Gly Lys Cys Gly Leu Val Pro Val Leu Ala Glu
            420                 425                 430
Asn Tyr Lys Thr Glu Gly Glu Ser Cys Lys Asn Thr Pro Glu Lys Gly
        435                 440                 445
Tyr Leu Ala Val Ala Val Val Lys Thr Ser Asp Ala Asn Ile Asn Trp
    450                 455                 460
Asn Asn Leu Lys Asp Lys Lys Ser Cys His Thr Ala Val Asp Arg Thr
465                 470                 475                 480
Ala Gly Trp Asn Ile Pro Met Gly Leu Leu Tyr Ser Lys Ile Asn Asn
                485                 490                 495
Cys Lys Phe Asp Glu Phe Phe Ser Ala Gly Cys Ala Pro Gly Ser Pro
            500                 505                 510
Arg Asn Ser Ser Leu Cys Ala Leu Cys Ile Gly Ser Glu Lys Gly Thr
        515                 520                 525

Gly Lys Glu Cys Val Pro Asn Ser Asn Glu Arg Tyr Tyr Gly Tyr Thr
    530                 535                 540
Gly Ala Phe Arg Cys Leu Val Glu Lys Gly Asp Val Ala Phe Val Lys
545                 550                 555                 560
Asp Gln Thr Val Ile Gln Asn Thr Asp Gly Asn Asn Asn Glu Ala Trp
                565                 570                 575
Ala Lys Asn Leu Lys Lys Glu Asn Phe Glu Val Leu Cys Lys Asp Gly
            580                 585                 590
Thr Arg Lys Pro Val Thr Asp Ala Glu Asn Cys His Leu Ala Arg Gly
        595                 600                 605
Pro Asn His Ala Val Val Ser Arg Lys Asp Lys Ala Thr Cys Val Glu
    610                 615                 620
Lys Ile Leu Asn Lys Gln Gln Asp Asp Phe Gly Lys Ser Val Thr Asp
625                 630                 635                 640
Cys Thr Ser Asn Phe Cys Leu Phe Gln Ser Asn Ser Lys Asp Leu Leu
                645                 650                 655
Phe Arg Asp Asp Thr Lys Cys Leu Ala Ser Ile Ala Lys Lys Thr Tyr
            660                 665                 670
Asp Ser Tyr Leu Gly Asp Asp Tyr Val Arg Ala Met Thr Asn Leu Arg
        675                 680                 685
Gln Cys Ser Thr Ser Lys Leu Leu Glu Ala Cys Thr Phe His Lys Pro
    690                 695                 700